# FASCICULE DE BREVET EUROPEEN

(11) **EP 0 908 175 B1**
(45) Date de publication et mention de la délivrance du brevet: **21.01.2004**
(21) Numéro de dépôt: 98402314.3
(22) Date de dépôt: 18.09.1998
(51) Int. Cl.: A61K 7/48

(54) **Composition topique stable contenant un organopolysiloxane élastomérique solide et des particules sphériques**
Stabile topische Zusammensetzungen mit einem festen elastomerischen Organopolysiloxan und sphärischen Partikeln
Stable topical composition containing a solid elastomeric organopolysiloxane and spherical particles

(30) Priorité: 01.10.1997 FR 9712223
(43) Date de publication de la demande: 14.04.1999
(73) Titulaire: L'OREAL, 75008 Paris (FR)
(72) Inventeur: Rouquet, Violaine, 06360 Eze (FR); Contamin, Jean-Claude, 06340 Cantaron (FR)
(74) Mandataire: Lhoste, Catherine

(56) Documents cités:
- EP-A- 0 829 253
- EP-A- 0 834 305
- WO-A-94/17774
- WO-A-97/32560
- US-A- 4 983 377
- US-A- 5 266 321

## Description

L'invention se rapporte à une composition topique stable, contenant une phase grasse liquide associée à un organopolysiloxane élastomérique partiellement ou totalement réticulé. Cette composition est plus spécialement destinée au domaine cosmétique ou dermatologique. Elle peut constituer notamment une composition de soin, de traitement, de maquillage ou de démaquillage de la peau du visage ou du corps, des fibres kératiniques (cheveux, cils, sourcils) et des muqueuses comme les lèvres et les surfaces internes des paupières, des êtres humains.

Il est connu d'utiliser dans les compositions cosmétiques ou dermatologiques des particules sphériques comme les particules de silice en vue de conférer une certaine consistance à ces compositions. On peut notamment se référer au document de Shiseido EP-A-765 656. Dans ces compositions, plus la quantité de particules est élevée, et plus la composition, est épaisse. En outre, ces particules ont la propriété d'absorber les matières grasses, conférant un aspect non gras à ces compositions, même en présence d'une grande quantité de matières grasses. Ce type de composition est bien apprécié des consommateurs et notamment des personnes de peaux à tendance grasse. Malheureusement, plus la quantité de particules de silice est élevée, et plus la composition est instable. En outre, ces particules confèrent à la composition un toucher très rêche et sec, limitant ainsi l'utilisation de ce type de composition.

Le demandeur a justement mis au point une composition stable à fort taux de particules sphériques ne présentant pas les inconvénients ci-dessus, tout en gardant la propriété de non gras.

De façon plus précise, l'invention se rapporte à une composition contenant au moins une phase grasse liquide associée à une phase solide comprenant des particules d'au moins un organopolysiloxane élastomérique partiellement ou totalement réticulé, caractérisée en ce que la phase solide représente au moins 10 % du poids total de la phase grasse et, contient, en outre, des particules sphériques organiques de diamètre de particules inférieur à 10 µm.

L'invention a aussi pour objet l'utilisation dans une composition, contenant au moins un phase grasse liquide et une phase solide représentant au moins 10 % du poids total de la phase grasse et contenant des particules sphériques organiques de diamètre de particules inférieur à 10 µm, de particules d'un ou plusieurs organopolysiloxanes élastomériques partiellement ou totalement réticulés, associées à ladite phase grasse, pour stabiliser et/ou rendre homogène ladite composition.

Elle a encore pour objet un procédé de stabilisation et/ou d'homogénéisation d'une composition contenant au moins une phase grasse liquide et une phase solide représentant au moins 10 % du poids total de la phase grasse et contenant des particules sphériques de diamètre de particules inférieur à 10 µm, consistant à associer à ladite phase grasse, des particules d'au moins un organopolysiloxane élastomérique partiellement ou totalement réticulé.

Grâce à la présence d'un ou plusieurs organopolysiloxanes solides élastomériques, il est possible d'obtenir des compositions stables contenant une quantité élevée de particules sphériques organiques pouvant représenter jusqu'à 40% en poids (en matière active) de la phase grasse totale.

Par "composition stable", il faut comprendre un maintien de l'aspect homogène de la composition, sans démixtion, précipitation ou floculation des particules, pendant au moins 2 mois à 45°C.

Par "élastomérique" on entend un matériau souple, déformable ayant des propriétés viscoélastiques et présentant notamment la consistance d'une éponge ou d'une sphère souple.

Par "phase grasse liquide", il faut comprendre une phase grasse liquide à température ambiante, souvent appelée phase huileuse.

Selon l'invention, il est possible, en outre, d'associer à la phase grasse liquide une phase grasse, solide ou semi-solide à température ambiante, en vue de modifier la rhéologie de la composition.

Bien que l'invention s'applique à tout domaine technique, elle est destinée plus spécialement aux domaines cosmétique et dermatologique. La composition de l'invention est bien adaptée à l'application topique.

Les organopolysiloxanes élastomériques de la composition selon l'invention présentent un remarquable pouvoir gélifiant d'huile. Ils ne sont pas desséchants pour ia peau et apportent de bonnes propriétés cosmétiques. Ces nouveaux élastomères conduisent à des compositions confortables à l'application, douces et non collantes au toucher. Cette douceur est due notamment à la texture des organopolysiloxanes.

L'association de l'invention permet également d'obtenir des produits de soin ou de maquillage destinés notamment à estomper les imperfections du relief de la peau tout en lui apportant un aspect naturel.

De préférence, les particules sphériques organiques ont un diamètre de particules inférieures à 5µm. Par diamètre de particules, il faut comprendre le diamètre des particules élémentaires. En effet, les particules sphériques peuvent avoir tendance à s'agglomérer conduisant à des agrégats pouvant avoir des diamètres de particules supérieurs à 5 µm, voire supérieurs à 10 µm.

En particulier, les particules sphériques organiques auxquelles s'applique l'invention, sont. des particules polymériques choisies parmi les microbilles de résines méthylsilsesquioxane comme par exemple celles vendues par Toshiba Silicone sous le nom Tospearl 145A, les microbilles de polyméthylméthacrylates comme notamment celles vendues par Seppic sous le nom Micropearl M 100, les particules sphériques de polydiméthylsiloxanes réticulés comme notamment celles vendues par Dow Corning Toray Silicone sous le nom Tréfil E 506 C ou Trefil E-505C, les particules sphériques de polyamide et plus spécialement de Nylon 12 comme notamment celles vendues par Atochem sous le nom Orgasol 2002 D Nat C05, les microsphères de polystyrène comme par exemple celles vendues par Dyno-Particles sous le nom Dynosphères, et leurs mélanges.

Les "Tréfil" sont en particulier des particules sphériques de polymères réticulés décrits dans la-demande EP-A-0295886 de Toray Silicone Company. Selon cette demande, ils sont obtenus par réaction d'addition et de réticulation, en présence d'un catalyseur du type platine, d'au moins :
- (a) un organopolysiloxane ayant au moins deux groupes alcényle inférieurs par molécule ; et
- (b) un organopolysiloxane ayant au moins deux atomes d'hydrogène liés à un atome de silicium par molécule.

Les organopolysiloxanes élastomériques de la composition de l'invention présentent une structure tridimensionnelle. Selon le taux de la phase grasse liquide associée à ces organopolysiloxanes, ces derniers se transforment d'un produit d'aspect spongieux lorsqu'ils sont utilisés en présence de faibles teneurs en phase grasse en un gel plus ou moins homogène, en présence de quantités de phase grasse plus élevées. La gélification de la phase grasse liquide par ces élastomères peut être totale ou partielle.

Les élastomères de la composition de l'invention sont véhiculés généralement sous forme de gel constitué d'un organopolysiloxane élastomérique de structure tridimensionnelle, inclus dans au moins une huile hydrocarbonée et/ou une huile siliconée.

Les organopolysiloxanes élastomériques de la composition selon l'invention peuvent aussi être choisis parmi ceux décrits dans le brevet US 5 266 321 de Kobayashi Kose. Selon ce brevet, ils sont choisis notamment parmi :
- i) les organopolysiloxanes comprenant des motifs R₂SiO et RSiO_{1.5} et éventuellement des motifs R₃SiO_{0.5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1.5} allant de 1/1 à 30/1 ;
- ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

Les organopolysiloxanes objet de l'invention sont par exemple ceux commercialisés sous les noms KSG6 de Shin-Etsu, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric. On peut aussi utiliser un mélange de ces produits commerciaux.

L'ensemble des particules d'organopolysiloxane élastomérique et des particules sphériques représente, au moins 10% (en matière active) du poids total de la phase grasse (liquide + solide) et mieux au moins 20% (en matière active). Cet ensemble peut, en outre, représenter jusqu'à 40% (en matière active) du poids total de la phase grasse.

La stabilité et l'homogénéité de la composition dépendent de la quantité de particules sphériques et croît avec la quantité d'organopolysiloxane élastomérique. A titre indicatif, le rapport en poids des particules sphériques et des particules d'organopolysiloxane élastomérique (en matière active) est choisi dans la gamme allant de 0,25 à 1 et mieux de 0,4 à 0,7. A titre préférentiel, la quantité de particules sphériques (en matière active) varie de 2% à 20 % du poids total de la composition et la quantité de particules d'organopolysiloxane élastomérique (en matière active) varie de 2% à 20% du poids total de la composition.

De façon avantageuse, la composition comprend une phase grasse continue. Grâce aux particules particulières présentent dans la composition, ce type de composition n'est ni gras au toucher et à l'application, ni huileux. En outre, cette composition est d'une grande douceur. Ainsi, la composition de l'invention peut être avantageusement un gel anhydre ou une émulsion eau-dans-huile (E/H). Toutefois, elle peut se présenter sous forme d'émulsion huile-dans-eau. Elle se présente, en outre, sous forme de crème.

Selon la quantité de particules utilisée, il est possible d'obtenir des compositions plus ou moins visqueuses, stables et homogènes au cours du temps. En particulier, la composition peut avoir une viscosité dynamique mesurée à température ambiante avec un appareil du type Rheomat RM 180 (Mettler) allant de 2 à 20 Pa.s.

Avantageusement, la composition de l'invention constitue une base de soin ou de maquillage, à appliquer sur la peau ou les lèvres avant le produit de soin ou de maquillage. Elle permet notamment de prolonger la tenue dans le temps de la composition de soin ou de maquillage, ce qui est particulièrement intéressant pour les produits de fond de teint, de camouflage des cernes, les brillants à lèvres (gloss en terminologie anglo-saxonne) et les produits de protection solaire. Elle peut également être utilisée comme composition matifiante de la peau, adaptée aux peaux grasses.

La phase grasse huileuse associée aux organopolysiloxanes élastomériques de l'invention peut comprendre des huiles hydrocarbonées et/ou des huiles siliconées.

Comme huiles hydrocarbonées utilisables dans l'invention, on peut citer notamment :
- les huiles hydrocarbonées d'origine animale telles que le perhydrosqualène ;
- les huiles hydrocarbonées d'origine végétale telles que les triglycérides liquides d'acides gras de 4 à 10 atomes de carbone comme les triglycérides des acides heptanoïque ou octanoïque, ou encore les huiles de tournesol, de maïs, de soja, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés d'origine minérale ou synthétique tels que les huiles de paraffine et leurs dérivés, les isoparaffines hydrogénées, volatiles ou non, la vaseline, les polydécènes, l'huile de Purcellin, le polyisobutène hydrogéné tel que le parléam ;
- les esters et les éthers de synthèse comme les huiles de formule R₁COOR₂ dans laquelle R, représente le reste d'un acide gras supérieur comportant de 6 à 29 atomes de carbone et R₂ représente une chaîne hydrocarbonée contenant de 3 à 30 atomes de carbone, telles que l'huile de Purcellin, le myristate d'isopropyle, le palmitate d'éthyl-2 hexyle, le stéarate d'octyl-2 dodécyle, l'isostéarate d'isostéaryle, le propionate d'arachidyle, le benzoate d'octyl-2 dodecyle ; les esters hydroxylés comme l'isostéaryl lactate, l'octylhydroxystéarate, l'hydroxystéarate d'octyldodécyle, le diisostéarylmalate, le citrate de triisocétyle ; les esters de polyol comme le dioctanoate de propylène glycol, le diheptanoate de néopentylglycol, le diisononanoate de diéthylèneglycol et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique, l'alcool cétylique ;
- leurs mélanges.

Les huiles siliconées utilisables dans l'invention sont notamment les polyméthylsiloxanes à structure linéaire ou cyclique, liquides ou pâteux à température ambiante, comme les polydiméthylsiloxanes tels que l'héxaméthyldisiloxane, l'octaméthylcyclopentasiloxane, le décaméthylcyclopentasiloxane, les phényl diméthicones, les phényl triméthicones et les polyméthylphénylsiloxanes, leurs mélanges.

La phase grasse solide ou semi-solide éventuellement présente dans la composition contient en particulier des cires et/ou des gommes. Les cires et les gommes utilisables dans l'invention sont notamment les cires microcristallines et les gommes de silicones.

La phase grasse totale peut représenter de 5 à 90 % du poids total du produit fini et mieux de 10 à 80 %.

La composition de l'invention contient avantageusement des matières colorantes et notamment une phase particulaire généralement présente à raison de 0,05 (voire 0%) à 35 % du poids total de la composition, de préférence de 2 à 25 %, et qui peut comprendre des pigments et/ou des nacres habituellement utilisés dans les produits cosmétiques. Cette phase peut conduire à un produit coloré, blanc ou incolore. Comme pigment utilisable dans la composition de l'invention, on peut citer les oxydes de titane, de zirconium ou de cérium ainsi que les oxydes de zinc, de fer ou de chrome, le bleu ferrique, le noir de carbone, et les laques de baryum, strontium, calcium, aluminium.

La composition selon l'invention peut comprendre, en outre, les ingrédients habituellement utilisés en cosmétique choisis en fonction de l'activité ou de l'effet cosmétique souhaité pour le produit final, tel que la couvrance, la transparence, la matité et/ou l'aspect satiné. On peut citer, sans effets limitatifs :
- les épaississants lipophiles ou hydrophiles comme les argiles modifiées connues sous les noms de bentone ; les sels gras d'aluminium ; la carboxyméthylcellulose ; les polyacrylamides ;
- les vitamines comme les tocophérols et leurs dérivés dont l'acétate, la vitamine A et ses dérivés, la vitamine C et ses dérivés comme les esters gras dont le palmitate ;
- les filtres solaires comme l'octylméthoxycinnamate (Parsol MCX), la 3-benzo-phénone (Uvinul M40), le butylméthoxydibenzoyl-méthane (Parsol 1789) ;
- la lécithine, les parfums, les huiles essentielles, les céramides, les conservateurs, les anti-oxydants ;
- les agents hydratants, tels que le propylène glycol, le glycérol ou même l'éthanol ;
- les agents agissant sur les peaux grasses et/ou anti-séborrhéiques tels que les sels de cuivre et/ou de zinc.

La composition peut également comprendre un tensioactif, par exemple un tensioactif usuel anionique ou non ionique. Le tensioactif est de préférence présent, à raison de 0,3 (voire 0% dans une composition anhydre) à 8% du poids total de la composition. Comme tensioactif utilisable dans la composition de l'invention, on peut citer le polysorbate 40, le monostéarate de glycérol, les diméthicone copolyols à chaîne oxypropylénée et/ou oxyéthylénée.

Les procédés de fabrication des compositions selon l'invention ne différent en rien des procédés classiquement utilisés en cosmétique et sont parfaitement connus de l'homme de l'art.

Dans les exemples ci-après, la quantité des ingrédients est donnée en pourcentage en poids.

### Exemple 1: Fond de teint E/H

| *Phase 1b :* | |
|---|---|
| Micropearl M 100 | 5 |
| | |

| *Phase 2b* : | |
|---|---|
| KSG 16 (à 24 % de siloxane réticulé) | 25,0 |
| | |

| *Phase grasse liquide :* | |
|---|---|
| Diméthicone Copolyol | 0,5 |
| Cyclométhicone | 4,5 |
| Octyl Méthoxycinnamate | 2,0 |
| PDMS liquide | 30,0 |
| Isoparaffine hydrogénée | 5,0 |
| Parfum | qs |
| | |

| *Phase colorante :* | |
|---|---|
| TiO2 | 4,0 |
| Oxydes de fer | 0,8 |
| | |

| *Phase aqueuse :* | |
|---|---|
| Glycérine | 10,0 |
| Eau déminéralisée | 15,0 |
| Conservateurs | qs |

Cette composition est obtenue en dispersant à température ambiante et sous agitation successivement les phases colorante, 1b puis 2b dans la phase liquide. Ensuite, on émulsionne dans une turbine la phase aqueuse dans la phase grasse à température ambiante. Cette composition a l'aspect d'une crème teintée, non grasse et douce. Elle présente une stabilité de 2 mois à 45 °C.

### Exemple 2 : Crème de soin H/E

| *Phase 1c :* | |
|---|---|
| Tréfil E 506C (100% matière active) | 2 |
| | |

| *Phase 2c* : | |
|---|---|
| Gransil SR 5 CYC (à 25 % de siloxane réticulé) | 8,0 |
| | |

| *Phase grasse liquide :* | |
|---|---|
| Polysorbate 40 | 2,0 |
| Monostéarate de glycérol | 2,0 |
| Alcool cétylique | 1,0 |
| Cyclométhicone | 2,0 |
| Diméthicone | 2,0 |
| Huile d'avocat | 2,0 |
| Huile de soja | 3,0 |
| Anti-oxydant (vitamine E) | 0,1 |
| | |

| *Phase aqueuse :* | |
|---|---|
| Glycérine | 5,0 |
| Polyacrylamide (gélifiant hydrophile) | 0,7 |
| Carboxyméthylcellulose | 0,5 |
| Eau déminéralisée | qsp 100 |
| Conservateur et parfum | qs |

On commence par émulsionner dans une turbine la phase aqueuse dans la phase grasse à 65°C. A 50 °C, on ajoute successivement les phases 1c puis 2c dans l'émulsion sous agitation dans une turbine. On laisse refroidir. Cette composition a l'aspect d'une crème blanche, non grasse et douce. Elle présente une stabilité de 2 mois à 45 °C.

### Exemple 3 : Test d'utilisation d'une base de maquillage

Cette composition est identique au fond de teint de l'exemple 1 sans la phase colorante.

| | |
|---|---|
| Personnes du test | 80 femmes, utilisatrices de fond de teint |
| Durée du test | 1 semaine |

La base de maquillage est appliquée avant le fond de teint et la comparaison est faite avec et sans base de maquillage.

Les résultats sont les suivants :

| | Sans Base (tout à fait d'accord) | Avec Base (tout à fait d'accord) |
|---|---|---|
| La peau est mate | 76 % | 94 % |
| La peau est douce | 49 % | 88 % |
| La peau est lisse | 40 % | 80 % |
| Le maquillage tient la journée | 74 % | 95 % |

De ce test, il ressort clairement que la composition de l'invention confère au maquillage des propriétés améliorées de tenue dans le temps, de confort, et de douceur.

## Revendications

1. Composition se présentant sous forme de crème, contenant au moins une phase grasse liquide associée à une phase solide comprenant des particules d'au moins un organopolysiloxane élastomérique partiellement ou totalement réticulé, **caractérisée en ce que** la phase solide représente au moins 10 % du poids total de la phase grasse et contient, en outre, des particules sphériques organiques de diamètre de particules inférieur à 10 µm, **en ce que** le rapport en poids des particules sphériques et des particules d'organopolysiloxane élastomérique est choisi dans la gamme allant de 0,25 à 1, **en ce que** la quantité de particules sphériques varie de 2% à 20 % du poids total de la composition, et **en ce que** l'organopolysiloxane élastomérique est de structure tridimensionnelle et est véhiculé sous forme de gel.

2. Composition selon la revendication 1, **caractérisée en ce que** l'organopolysiloxane est obtenu par réaction d'addition et de réticulation, en présence d'un catalyseur de type platine :
a) d'au moins un organopolysiloxane ayant au moins deux groupes alcényle inférieurs par molécule ; et
(b) d'au moins un organopolysiloxane ayant au moins deux atomes d'hydrogène liés à un atome de silicium par molécule.

3. Composition selon la revendication 1 ou 2, **caractérisée en ce que** l'organopolysiloxane est choisi parmi :
i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle. tel que phényle ou tolyle, un groupe aliphatique insaturé tel que vinyle et où le rapport en poids des motifs R₂SiO sur les motifs RSiO_{1,5} varie de 1/1 à 30/1 ;
ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

4. Composition selon l'une des revendications précédentes, **caractérisée en ce que** les particules sphériques ont un diamètre de particules inférieur à 5µm.

5. Composition selon l'une des revendications précédentes, **caractérisée en ce que** les particules sphériques sont choisies parmi les microbilles de résines méthylsilsesquioxane, les microbilles de polyméthacrylates de méthyle, les particules sphériques de polydiméthylsiloxanes réticulés, les particules sphériques de polyamide, les microsphères de polystyrène et leurs mélanges.

6. Composition selon l'une des revendications précédentes, **caractérisée en ce que** les particules sphériques sont choisies parmi les particules organiques polymériques.

7. Composition selon l'une des revendications précédentes, **caractérisée en ce que** la phase solide représente au moins 20 % du poids total de la phase grasse.

8. Composition selon l'une des revendications précédentes, **caractérisée en ce que** le rapport en poids des particules sphériques et des particules d'organopolysiloxane élastomérique est choisi dans la gamme allant de 0,4 à 0,7.

9. Composition selon l'une des revendications précédentes, **caractérisée en ce que** la phase grasse liquide contient au moins une huile siliconée et/ou au moins une huile hydrocarbonée.

10. Composition selon l'une des revendications précédentes, **caractérisée en ce que** la phase grasse liquide contient au moins une huile choisie parmi les huiles hydrocarbonées d'origine animale, végétale, minérale, de synthèse, les alcools gras, les polyméthylsiloxanes.

11. Composition selon l'une des revendications précédentes, **caractérisée en ce que** la composition contient, en outre une phase grasse solide ou semi-solide.

12. Composition selon l'une des revendications précédentes, **caractérisée en ce que** la composition se présente sous la forme d'un gel anhydre, d'une émulsion eau-dans-huile ou huile-dans-eau.

13. Composition selon l'une des revendications précédentes, **caractérisée en ce que** la composition comprend une phase grasse continue.

14. Composition selon l'une des revendications précédentes, **caractérisée en ce qu'**elle se présente sous forme de composition cosmétique ou dermatologique.

15. Composition selon l'une des revendications précédentes, **caractérisée en ce que** la composition se présente sous la forme d'une composition de soin, de traitement, de maquillage ou de démaquillage de la peau, des muqueuses ou des fibres kératiniques.

16. Composition selon l'une des revendications précédentes, **caractérisée en ce qu'**elle contient, en outre, un ingrédient choisi parmi les filtres solaires, les huiles essentielles, les vitamines, les agents antiséborrhéiques, les extraits marins, les émollients, les antioxydants les épaississants hydrophiles, les épaississants lipophiles, les conservateurs, les parfums, les matières colorantes et leurs mélanges.

17. Composition selon l'une des revendications précédentes, **caractérisée en ce qu'**elle constitue une base de soin ou de maquillage de la peau ou des lèvres.

18. Utilisation de la composition conforme à l'une des revendications précédentes, pour estomper les imperfections du relief de la peau et/ou prolonger la tenue dans le temps de la composition et/ou matifier la peau.

19. Utilisation dans une composition sous forme de crème, contenant au moins un phase grasse liquide et une phase solide représentant au moins 10 % du poids total de la phase grasse et contenant des particules sphériques organiques de diamètre de particules inférieur à 10 µm, de particules d'au moins un organopolysiloxane élastomérique partiellement ou totalement réticulé associées à ladite phase grasse, le rapport en poids des particules sphériques et des particules d'organopolysiloxane élastomérique étant choisi dans la gamme allant de 0,25 à 1, la quantité de particules sphériques variant de 2% à 20 % du poids total de la composition et l'organopolysiloxane élastomérique étant de structure tridimensionnelle et étant véhiculé sous forme de gel, pour stabiliser et/ou rendre homogène ladite composition.

20. Utilisation selon la revendication 19, **caractérisée en ce que** l'organopolysiloxane est obtenu par réaction d'addition et de réticulation, en présence d'un catalyseur de type platine :
a) d'au moins un organopolysiloxane ayant au moins deux groupes alcényle inférieurs par molécule ; et
(b) d'au moins un organopolysiloxane ayant au moins deux atomes d'hydrogène liés à un atome de silicium par molécule.

21. Utilisation selon la revendication 19 ou 20, **caractérisée en ce que** l'organopolysiloxane est choisi parmi :
i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs R₂SiO sur les motifs RSiO_{1,5} varie de 1/1 à 30/1 ;
ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

22. Utilisation selon l'une des revendications 19 à 21, **caractérisée en ce que** le rapport en poids des particules sphériques et de l'organopolysiloxane élastomérique est choisi dans la gamme allant de 0,4 à 0,7.

23. Utilisation selon l'une des revendications 19 à 22, **caractérisée en ce que** la phase grasse liquide contient au moins une huile siliconée et/ou au moins une huile hydrocarbonée.

24. Utilisation selon l'une des revendications 19 à 23, **caractérisée en ce que** la composition constitue une base de soin ou de maquillage de la peau ou des lèvres.

25. Procédé de stabilisation et/ou d'homogénéisation d'une composition sous forme de crème, contenant au moins une phase grasse liquide et une phase solide représentant au moins 10 % du poids total de la phase grasse et contenant des particules sphériques de diamètre de particules inférieur à 10 µm, consistant à associer à ladite phase grasse, des particules d'au moins un organopolysiloxane élastomérique partiellement ou totalement réticulé le rapport en poids des particules sphériques et des particules d'organopolysiloxane élastomérique étant choisi dans la gamme allant de 0,25 à 1, la quantité de particules sphériques variant de 2% à 20 % du poids total de la composition, l'organopolysiloxane élastomérique étant de structure tridimensionnelle et étant véhiculé sous forme de gel.

26. Procédé selon la revendication 25, **caractérisé en ce que** l'organopolysiloxane est choisi parmi :
i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs R₂SiO sur les motifs RSiO_{1,5} varie de 1/1 à 30/1 ;
ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

27. Procédé selon l'une des revendications 25 à 26, **caractérisé en ce que** le rapport en poids des particules sphériques et de l'organopolysiloxane élastomérique est choisi dans la gamme allant de 0,4 à 0,7.

## Patentansprüche

1. Zusammensetzung, die in Form einer Creme vorliegt und mindestens eine flüssige Fettphase in Kombination mit einer festen Phase enthält, welche Partikel mindestens eines ganz oder teilweise vernetzten, elastomeren Organopolysiloxans enthält, **dadurch gekennzeichnet, dass** die feste Phase mindestens 10 % des Gesamtgewichts der Fettphase ausmacht und ferner organische sphärische Partikel mit einem Partikeldurchmesser unter 10 µm enthält, dadurch, dass das Gewichtsverhältnis der sphärischen Partikel und der Partikel des elastomeren Organopolysiloxans im Bereich von 0,25 bis 1 ausgewählt ist, dadurch, dass der Mengenanteil der sphärischen Partikel im Bereich von 2 bis 20 % des Gesamtgewichts der Zusammensetzung liegt, und dadurch, dass das elastomere Organopolysiloxan eine dreidimensionale Struktur besitzt und in Form eines Gels bereitgestellt wird.

2. Zusammensetzung nach Anspruch 1, **dadurch gekennzeichnet, dass** das Organopolysiloxan in Gegenwart eines Katalysators vom Platintyp durch Addition und Vernetzung der folgenden Verbindungen hergestellt wird:
(a) mindestens eines Organopolysiloxans mit mindestens zwei niederen Alkenylgruppen pro Molekül; und
(b) mindestens eines Organopolysiloxans mit mindestens zwei Wasserstoffatomen pro Molekül, die an ein Siliciumatom gebunden sind.

3. Zusammensetzung nach Anspruch 1 oder 2, **dadurch gekennzeichnet, dass** das Organopolysiloxan ausgewählt ist unter:
i) Organopolysiloxanen mit Einheiten R₂SiO und RSiO_{1,5} und ggf. Einheiten R₃SiO_{0,5} und/ oder SiO₂, worin die Gruppen R unabhängig voneinander Wasserstoff, eine Alkylgruppe, wie Methyl, Ethyl oder Propyl, eine Arylgruppe, wie Phenyl oder Tolyl, oder eine ungesättigte aliphatische Gruppe, wie Vinyl, bedeuten, wobei das Gewichtsverhältnis der Einheiten R₂SiO und der Einheiten RSiO_{1,5} im Bereich von 1/1 bis 30/1 liegt;
ii) unlöslichen und in Siliconöl quellfähigen Organopolysiloxanen, die durch Addition eines Organohydrogenpolysiloxans (1) und eines Organopolysiloxans (2) mit ungesättigten aliphatischen Gruppen so hergestellt werden, dass die Wasserstoffmenge oder die Menge der ungesättigten aliphatischen Gruppen in (1) bzw. (2) im Bereich von 1 bis 20 Mol-% liegt, falls das Organopolysiloxan nicht cyclisch ist, und im Bereich von 1 bis 50 Mol-% liegt, falls das Organopolysiloxan cyclisch vorliegt.

4. Zusammensetzung nach einem der vorhergehenden Ansprüche, **dadurch gekennzeichnet, dass** die sphärischen Partikel einen Partikeldurchmesser unter 5 µm besitzen.

5. Zusammensetzung nach einem der vorhergehenden Ansprüche, **dadurch gekennzeichnet, dass** die sphärischen Partikel unter den Mikrokugeln von Methylsilsesquioxanharzen, Mikrokugeln von Polymethylmethacrylaten, sphärischen Partikeln von vernetzten Polydimethylsiloxanen, sphärischen Partikeln von Polyamid, Polystyrolmikrosphären und deren Gemischen ausgewählt sind.

6. Zusammensetzung nach einem der vorhergehenden Ansprüche, **dadurch gekennzeichnet, dass** die sphärischen Partikel unter den polymeren organischen Partikeln ausgewählt sind.

7. Zusammensetzung nach einem der vorhergehenden Ansprüche, **dadurch gekennzeichnet, dass** die feste Phase mindestens 20 % des Gesamtgewichts der Fettphase ausmacht.

8. Zusammensetzung nach einem der vorhergehenden Ansprüche, **dadurch gekennzeichnet, dass** das Gewichtsverhältnis der sphärischen Partikel und der Partikel des elastomeren Organopolysiloxans im Bereich von 0,4 bis 0,7 liegt.

9. Zusammensetzung nach einem der vorhergehenden Ansprüche, **dadurch gekennzeichnet, dass** die flüssige Fettphase mindestens ein Siliconöl und/oder mindestens ein Kohlenwasserstofföl enthält.

10. Zusammensetzung nach einem der vorhergehenden Ansprüche, **dadurch gekennzeichnet, dass** die flüssige Fettphase mindestens ein Öl enthält, das unter den Kohlenwasserstoffölen tierischer, pflanzlicher, mineralischer oder synthetischer Herkunft, Fettalkoholen und Polymethylsiloxanen ausgewählt ist.

11. Zusammensetzung nach einem der vorhergehenden Ansprüche, **dadurch gekennzeichnet, dass** die Zusammensetzung ferner eine feste oder halbfeste Fettphase enthält.

12. Zusammensetzung nach einem der vorhergehenden Ansprüche, **dadurch gekennzeichnet, dass** die Zusammensetzung in Form eines wasserfreien Gels, einer Wasser-in-Öl-Emulsion oder einer Öl-in-Wasser-Emulsion vorliegt.

13. Zusammensetzung nach einem der vorhergehenden Ansprüche, **dadurch gekennzeichnet, dass** die Zusammensetzung eine kontinuierliche Fettphase enthält.

14. Zusammensetzung nach einem der vorhergehenden Ansprüche, **dadurch gekennzeichnet, dass** sie in Form einer kosmetischen oder dermatologischen Zusammensetzung vorliegt.

15. Zusammensetzung nach einem der vorhergehenden Ansprüche, **dadurch gekennzeichnet, dass** die Zusammensetzung in Form einer Zusammensetzung zur Pflege, zur Behandlung, zum Schminken oder zum Abschminken der Haut, der Schleimhäute oder der Keratinfasern vorliegt.

16. Zusammensetzung nach einem der vorhergehenden Ansprüche, **dadurch gekennzeichnet, dass** sie ferner einem Bestandteil enthält, der unter den Sonnenschutzfiltern, etherischen Ölen, Vitaminen, Antiseborrhoeika, marinen Extrakten, Emollientien, Antioxidantien, hydrophilen Verdickungsmitteln, lipophilen Verdickungsmitteln, Konservierungsmitteln, Parfums, Farbmitteln und deren Gemischen ausgewählt ist.

17. Zusammensetzung nach einem der vorhergehenden Ansprüche, **dadurch gekennzeichnet, dass** es sich um eine Basisformulierung zur Pflege oder zum Schminken der Haut oder der Lippen handelt.

18. Verwendung der Zusammensetzung nach einem der vorhergehenden Ansprüche, um die Unebenheiten des Hautreliefs zu kaschieren und/oder die zeitliche Haftung der Zusammensetzung zu verbessern und/oder die Haut zu mattieren.

19. Verwendung von Partikeln mindestens eines ganz oder teilweise vernetzten, elastomeren Organopolysiloxans in Kombination mit einer Fettphase in einer Zusammensetzung in Cremeform, die mindestens eine flüssige Fettphase und eine feste Phase enthält, welche mindestens 10 % des Gesamtgewichts der Fettphase ausmacht und organische sphärische Partikel mit einem Partikeldurchmesser unter 10 µm enthält, wobei das Gewichtsverhältnis der sphärischen Partikel und der Partikel des elastomeren Organopolysiloxans im Bereich von 0,25 bis 1 ausgewählt ist, der Mengenanteil der sphärischen Partikel im Bereich von 2 bis 20 % des Gesamtgewichts der Zusammensetzung liegt, das elastomere Organopolysiloxan eine dreidimensionale Struktur besitzt und in Gelform bereitgestellt wird, zur Stabilisierung und/oder Homogenisierung der Zusammensetzung.

20. Verwendung nach Anspruch 19, **dadurch gekennzeichnet, dass** das Organopolysiloxan in Gegenwart eines Katalysators vom Platintyp durch Addition und Vernetzung der folgenden Verbindungen hergestellt wird:
(a) mindestens eines Organopolysiloxans mit mindestens zwei niederen Alkenylgruppen pro Molekül; und
(b) mindestens eines Organopolysiloxans mit mindestens zwei Wasserstoffatomen pro Molekül, die an ein Siliciumatom gebunden sind.

21. Verwendung nach Anspruch 19 oder 20, **dadurch gekennzeichnet, dass** das Organopolysiloxan ausgewählt ist unter:
i) Organopolysiloxanen mit Einheiten R₂SiO und RSiO_{1,5} und ggf. Einheiten R₃SiO_{0,5} und/oder SiO₂, worin die Gruppen R unabhängig voneinander Wasserstoff, eine Alkylgruppe, wie Methyl, Ethyl oder Propyl, eine Arylgruppe, wie Phenyl oder Tolyl, oder eine ungesättigte aliphatische Gruppe, wie Vinyl, bedeuten, wobei das Gewichtsverhältnis der Einheiten R₂SiO und der Einheiten RSiO_{1,5} im Bereich von 1/1 bis 30/1 liegt;
ii) unlöslichen und in Siliconöl quellfähigen Organopolysiloxanen, die durch Addition eines Organohydrogenpolysiloxans (1) und eines Organopolysiloxans (2) mit ungesättigten aliphatischen Gruppen so hergestellt werden, dass die Wasserstoffmenge oder die Menge der ungesättigten aliphatischen Gruppen in (1) bzw. (2) im Bereich von 1 bis 20 Mol-% liegt, falls das Organopolysiloxan nicht cyclisch ist, und im Bereich von 1 bis 50 Mol-%, falls das Organopolysiloxan cyclisch vorliegt.

22. Verwendung nach einem der Ansprüche 19 bis 21, **dadurch gekennzeichnet, dass** das Gewichtsverhältnis der sphärischen Partikel und der Partikel des elastomeren Organopolysiloxans im Bereich von 0,4 bis 0,7 liegt.

23. Verwendung nach einem der Ansprüche 19 bis 22, **dadurch gekennzeichnet, dass** die flüssige Fettphase mindestens ein Siliconöl und/oder mindestens ein Kohlenwasserstofföl enthält.

24. Verwendung nach einem der Ansprüche 19 bis 23, **dadurch gekennzeichnet, dass** die Zusammensetzung eine Basisformulierung zur Pflege oder zum Schminken der Haut oder der Lippen ist.

25. Verfahren zur Stabilisierung und/oder Homogenisierung einer Zusammensetzung in Cremeform, die mindestens eine flüssige Fettphase und eine feste Phase enthält, welche mindestens 10 % des Gesamtgewichts der Fettphase ausmacht und sphärische Partikel mit einem Partikeldurchmesser unter 10 µm enthält, das darin besteht, mit der Fettphase Partikel mindestens eines ganz oder teilweise vernetzten, elastomeren Organopolysiloxans zu kombinieren, wobei das Gewichtsverhältnis der sphärischen Partikel und der Partikel des elastomeren Organopolysiloxans im Bereich von 0,25 bis 1 ausgewählt wird, der Mengenanteil der sphärischen Partikel im Bereich von 2 bis 20 % des Gesamtgewichts der Zusammensetzung liegt, das elastomere Organopolysiloxan eine dreidimensionale Struktur besitzt und in Gelform bereitgestellt wird.

26. Verfahren nach Anspruch 25, **dadurch gekennzeichnet, dass** das Organopolysiloxan ausgewählt ist unter:
i) Organopolysiloxanen mit Einheiten R₂SiO und RSiO_{1,5} und ggf. Einheiten R₃SiO_{0,5} und/oder SiO₂, worin die Gruppen R unabhängig voneinander Wasserstoff, eine Alkylgruppe, wie Methyl, Ethyl oder Propyl, eine Arylgruppe, wie Phenyl oder Tolyl, oder eine ungesättigte aliphatische Gruppe, wie Vinyl, bedeuten, wobei das Gewichtsverhältnis der Einheiten R₂SiO und der Einheiten RSiO_{1,5} im Bereich von 1/1 bis 30/1 liegt;
ii) unlöslichen und in Siliconöl quellfähigen Organopolysiloxanen, die durch Addition eines Organohydrogenpolysiloxans (1) und eines Organopolysiloxans (2) mit ungesättigten aliphatischen Gruppen so hergestellt werden, dass die Wasserstoffmenge oder die Menge der ungesättigten aliphatischen Gruppen in (1) bzw. (2) im Bereich von 1 bis 20 Mol-% liegt, falls das Organopolysiloxan nicht cyclisch ist, und im Bereich von 1 bis 50 Mol-%, falls das Organopolysiloxan cyclisch vorliegt.

27. Verfahren nach einem der Ansprüche 25 bis 26, **dadurch gekennzeichnet, dass** das Gewichtsverhältnis der sphärischen Partikel und der Partikel des elastomeren Organopolysiloxans im Bereich von 0,4 bis 0,7 ausgewählt wird.

## Claims

1. Composition provided in the form of a cream, comprising at least one liquid fatty phase in combination with a solid phase comprising particles of at least one partially or completely crosslinked elastomeric organopolysiloxane, **characterized in that** the solid phase represents at least 10% of the total weight of the fatty phase and, in addition, comprises spherical organic particles with a particle diameter of less than 10 µm, **in that** the ratio by weight of the spherical particles to the elastomeric organopolysiloxane particles is chosen within the range from 0.25 to 1, **in that** the amount of spherical particles varies from 2% to 20% of the total weight of the composition and **in that** the elastomeric organopolysiloxane has a three-dimensional structure and is carried in the form of a gel.

2. Composition according to Claim 1, **characterized in that** the organopolysiloxane is obtained by addition and crosslinking reaction, in the presence of a catalyst of platinum type:
(a) of at least one organopolysiloxane having at least two lower alkenyl groups per molecule; and
(b) of at least one organopolysiloxane having at least two hydrogen atoms bonded to a silicon atom per molecule.

3. Composition according to Claim 1 or 2, **characterized in that** the organopolysiloxane is chosen from:
i) organopolysiloxanes comprising R₂SiO and RSiO_{1.5} units and optionally R₃SiO_{0.5} and/or SiO₂ units in which the R radicals, independently of one another, denote a hydrogen, an alkyl, such as methyl, ethyl or propyl, an aryl, such as phenyl or tolyl, or an unsaturated aliphatic group, such as vinyl, and where the ratio by weight of the R₂SiO units to the RSiO_{1.5} units varies from 1/1 to 30/1;
ii) organopolysiloxanes which are insoluble and which can be swollen in a silicone oil, obtained by addition of an organohydropolysiloxane (1) and of an organopolysiloxane (2) having unsaturated aliphatic groups, so that the amount of hydrogen or of unsaturated aliphatic groups in (1) and (2) respectively is between 1 and 20 mol % when the organopolysiloxane is non-cyclic and between 1 and 50 mol % when the organopolysiloxane is cyclic.

4. Composition according to one of the preceding claims, **characterized in that** the spherical particles have a particle diameter of less than 5 µm.

5. Composition according to one of the preceding claims, **characterized in that** the spherical particles are chosen from microbeads of methylsilsesquioxane resins, microbeads of poly(methyl methacrylate)s, spherical particles of crosslinked polydimethylsiloxanes, spherical particles of polyamide, polystyrene microspheres and their mixtures.

6. Composition according to one of the preceding claims, **characterized in that** the spherical particles are chosen from polymeric organic particles.

7. Composition according to one of the preceding claims, **characterized in that** the solid phase represents at least 20% of the total weight of the fatty phase.

8. Composition according to one of the preceding claims, **characterized in that** the ratio by weight of the spherical particles to the elastomeric organopolysiloxane particles is chosen within the range from 0.4 to 0.7.

9. Composition according to one of the preceding claims, **characterized in that** the liquid fatty phase comprises at least one silicone oil and/or at least one hydrocarbon-comprising oil.

10. Composition according to one of the preceding claims, **characterized in that** the liquid fatty phase comprises at least one oil chosen from hydrocarbon-comprising oils of animal, plant, mineral or synthetic origin, fatty alcohols or polymethylsiloxanes.

11. Composition according to one of the preceding claims, **characterized in that** the composition additionally comprises a solid or semi-solid fatty phase.

12. Composition according to one of the preceding claims, **characterized in that** the composition is provided in the form of an anhydrous gel or of a water-in-oil or oil-in-water emulsion.

13. Composition according to one of the preceding claims, **characterized in that** the composition comprises a continuous fatty phase.

14. Composition according to one of the preceding claims, **characterized in that** it is provided in the form of a cosmetic or dermatological composition.

15. Composition according to one of the preceding claims, **characterized in that** the composition is provided in the form of a composition for caring for, treating, making up or removing make-up from the skin, mucous membranes or keratinous fibres.

16. Composition according to one of the preceding claims, **characterized in that** it additionally comprises an ingredient chosen from sunscreening agents, essential oils, vitamins, antiseborrhoeic agents, marine extracts, emollients, antioxidants, hydrophilic thickeners, lipophilic thickeners, preservatives, fragrances, colouring materials and their mixtures.

17. Composition according to one of the preceding claims, **characterized in that** it constitutes a care or make-up base for the skin or the lips.

18. Use of the composition in accordance with one of the preceding claims for softening blemishes of the contours of the skin and/or extending the hold over time of the composition and/or rendering the skin matt.

19. Use, in a composition in the form of a cream comprising at least one liquid fatty phase and one solid phase representing at least 10% of the total weight of the fatty phase and comprising spherical organic particles with a particle diameter of less than 10 µm, of particles of at least one partially or completely crosslinked elastomeric organopolysiloxane, in combination with the said fatty phase, the ratio by weight of the spherical particles to the elastomeric organopolysiloxane particles being chosen within the range from 0.25 to 1, the amount of spherical particles varying from 2% to 20% of the total weight of the composition and the elastomeric organopolysiloxane having a three-dimensional structure and being carried in the form of a gel, in order to stabilize and/or render homogeneous the said composition.

20. Use according to Claim 19, **characterized in that** the organopolysiloxane is obtained by addition and crosslinking reaction, in the presence of a catalyst of platinum type:
(a) of at least one organopolysiloxane having at least two lower alkenyl groups per molecule; and
(b) of at least one organopolysiloxane having at least two hydrogen atoms bonded to a silicon atom per molecule.

21. Use according to Claim 19 or 20, **characterized in that** the organopolysiloxane is chosen from:
i) organopolysiloxanes comprising R₂SiO and RSiO_{1.5} units and optionally R₃SiO_{0.5} and/or SiO₂ units in which the R radicals, independently of one another, denote a hydrogen, an alkyl, such as methyl, ethyl or propyl, an aryl, such as phenyl or tolyl, or an unsaturated aliphatic group, such as vinyl, and where the ratio by weight of the R₂SiO units to the RSiO_{1.5} units varies from 1/1 to 30/1;
ii) organopolysiloxanes which are insoluble and which can be swollen in a silicone oil, obtained by addition of an organohydropolysiloxane (1) and of an organopolysiloxane (2) having unsaturated aliphatic groups, so that the amount of hydrogen or of unsaturated aliphatic groups in (1) and (2) respectively is between 1 and 20 mol % when the organopolysiloxane is non-cyclic and between 1 and 50 mol % when the organopolysiloxane is cyclic.

22. Use according to one of Claims 19 to 21, **characterized in that** the ratio by weight of the spherical particles to the elastomeric organopolysiloxane particles is chosen within the range from 0.4 to 0.7.

23. Use according to one of Claims 19 to 22, **characterized in that** the liquid fatty phase comprises at least one silicone oil and/or at least one hydrocarbon-comprising oil.

24. Use according to one of Claims 19 to 23, **characterized in that** the composition constitutes a care or make-up base for the skin or lips.

25. Process for the stabilization and/or homogenization of a composition in the form of a cream comprising at least one liquid fatty phase and one solid phase representing at least 10% of the total weight of the fatty phase and comprising spherical particles with a particle diameter of less than 10 µm, which consists in combining, with the said fatty phase, particles of at least one partially or completely crosslinked elastomeric organopolysiloxane, the ratio by weight of the spherical particles to the elastomeric organopolysiloxane particles being chosen within the range from 0.25 to 1, the amount of spherical particles varying from 2% to 20% of the total weight of the composition, the elastomeric organopolysiloxane having a three-dimensional structure and being carried in the form of a gel

26. Process according to Claim 25, **characterized in that** the organopolysiloxane is chosen from:
i) organopolysiloxanes comprising R₂SiO and RSiO_{1.5} units and optionally R₃SiO_{0.5} and/or SiO₂ units in which the R radicals, independently of one another, denote a hydrogen, an alkyl, such as methyl, ethyl or propyl, an aryl, such as phenyl or tolyl, or an unsaturated aliphatic group, such as vinyl, and where the ratio by weight of the R₂SiO units to the RSiO_{1.5} units varies from 1/1 to 30/1;
ii) organopolysiloxanes which are insoluble and which can be swollen in a silicone oil, obtained by addition of an organohydropolysiloxane (1) and of an organopolysiloxane (2) having unsaturated aliphatic groups, so that the amount of hydrogen or of unsaturated aliphatic groups in (1) and (2) respectively is between 1 and 20 mol % when the organopolysiloxane is non-cyclic and between 1 and 50 mol % when the organopolysiloxane is cyclic.

27. Process according to one of Claims 25 to 26, **characterized in that** the ratio by weight of the spherical particles to the elastomeric organopolysiloxane particles is chosen within the range from 0.4 to 0.7.
